(19)

Europäisches
Patentamt
European
Patent Office
Office européen
des brevets

(11)  **EP 3 108 797 A1**

(12)  **EUROPEAN PATENT APPLICATION**
published in accordance with Art. 153(4) EPC

(43) Date of publication:
**28.12.2016  Bulletin 2016/52**

(21) Application number: **15752046.1**

(22) Date of filing: **18.02.2015**

(51) Int Cl.:
**A61B 1/00** *(2006.01)*      **G02B 23/24** *(2006.01)*

(86) International application number:
**PCT/JP2015/054355**

(87) International publication number:
**WO 2015/125796 (27.08.2015 Gazette 2015/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority:  **20.02.2014   JP 2014030516**

(71) Applicant: **Olympus Corporation
Hachioji-shi
Tokyo 192-8507 (JP)**

(72) Inventors:
• **HATAKEYAMA, Naoya
Hachioji-shi
Tokyo 192-8507 (JP)**

• **IIDA, Masatoshi
Hachioji-shi
Tokyo 192-8507 (JP)**
• **WAKAI, Hiroshi
Hachioji-shi
Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(54)  **MANIPULATOR SYSTEM AND CONTROL METHOD THEREFOR**

(57)      Even in the case in which an inserted portion is bent in a complicated manner, a manipulator at the distal end thereof is controlled with high precision. Provided is a manipulator system including: a flexible inserted portion (10); a manipulator (6) that has a joint portion that is driven at the distal end of the inserted portion (10); a drive portion (7) that drives the joint portion of the manipulator (6) on the basal-end side of the inserted portion (10); a shape estimating portion that estimates the shape of the inserted portion (10); and a control portion that controls the drive portion (7) on the basis of the shape of the inserted portion (10) estimated by the shape estimating portion, wherein the shape estimating portion is provided with a plurality of detection targets (14) that are arranged, with spaces therebetween, in the inserted portion (10) in the longitudinal direction thereof, a position detection portion (15) that detects three-dimensional positions of the detection targets (14), and a shape calculating portion that calculates, on the basis of the three-dimensional positions of the detection targets (14) detected by the position detection portion (15), the shape of the inserted portion (10) by dividing the inserted portion (10) into a plurality of sections (SS) in the longitudinal direction thereof.

FIG. 2

**Description**

{Technical Field}

The present invention relates to a manipulator system and a method of controlling the same.

{Background Art}

In the related art, there is a known endoscope system in which a UPD device is used to detect the shape of a bending portion of an endoscope, and the bending portion is controlled by using the detected shape thereof (for example, see Patent Literature 1).

{Citation List}

{Patent Literature}

{PTL 1} Japanese Unexamined Patent Application, Publication No. 2009-131406

{Summary of Invention}

{Technical Problem}

However, in a flexible endoscope, because not only the bending portion but also an inserted portion, which is connected at the basal-end side of the bending portion, changes its shape in a complicated manner in accordance with the insertion route, there is a problem with the endoscope system of Patent Literature 1, which detects and uses the shape of the bending portion, in that it is difficult to control the bending portion with high precision.

The present invention has been conceived in light of the above-described circumstances and provides a manipulator system and a method of controlling the same with which it is possible to control a manipulator at the distal end with high precision, even when an inserted portion is bent in a complicated manner.

{Solution to Problem}

In order to achieve the above-described object, the present invention provides the following solutions.

An aspect of the present invention is a manipulator system including: a flexible inserted portion; a manipulator that has a joint portion that is driven at a distal end of the inserted portion; a drive portion that drives the joint portion of the manipulator; a shape estimating portion that estimates the shape of the inserted portion; and a control portion that controls the drive portion on the basis of the shape of the inserted portion estimated by the shape estimating portion, wherein the shape estimating portion is provided with a plurality of detection targets that are arranged, with spaces therebetween, in the inserted portion in the longitudinal direction thereof, a position detection portion that detects three-dimensional positions of the individual detection targets, and a shape calculating portion that calculates, on the basis of the three-dimensional positions of the individual detection targets detected by the position detection portion, the shape of the inserted portion by dividing the inserted portion into a plurality of sections that are arranged in the longitudinal direction thereof.

With this aspect, when the inserted portion is inserted into the body cavity from the distal-end side, the shape of the inserted portion changes so as to conform to the shape of the body cavity. At this time, because the detection targets disposed in the inserted portion are arranged so as to be dispersed with spaces therebetween in the longitudinal direction of the inserted portion, by detecting the three-dimensional positions of the individual detection targets by using the position detection portion, it is possible to estimate the shape of the inserted portion along the longitudinal direction of the inserted portion by using the shape estimating portion.

In this case, because the shape calculating portion calculates the shape of the inserted portion by dividing the inserted portion into the plurality of sections arranged in the longitudinal direction, it is possible to calculate, with high precision, the shape of the inserted portion in which the overall shape thereof has been deformed in a complicated manner, even if estimation in each section is performed by using a simple shape. As a result, it is possible to control, with high precision by using the control portion, driving of the manipulator at the distal end of the inserted portion by the drive portion disposed on the basal-end side of the inserted portion on the basis of the estimated shape of the inserted portion.

In the above-described aspect, the shape calculating portion may set each of the sections so as to be located between two of the detection targets that are adjacent to each other.

By doing so, it is possible to set a maximum number of sections, and thus, it is possible to estimate a complicated shape of the inserted portion as a whole even if the shape of the inserted portion in each section is estimated by using a simple shape.

In the above-described aspect, the shape calculating portion may set each of the sections so as to include three of the detection targets that are adjacent to each other.

By doing so, it is possible to estimate the shape of the inserted portion with high precision by using a curve that passes through the three detection targets.

In the above-described aspect, the shape calculating portion may approximate, for each of the sections, a part between two of the detection targets that are adjacent to each other by using a single arc.

By doing so, it is possible to effectively estimate, for each of the sections that are set so as to include two detection targets each, the shape of the inserted portion in a simple manner by using the arc that passes through the two detection targets.

In the above-described aspect, the shape calculating portion may approximate, for each of the sections, a part across three of the adjacent detection targets by using a single arc.

By doing so, it is possible to effectively estimate, for each of the sections that are set so as to include three detection targets each, the shape of the inserted portion in a simple manner by using the arc that passes through the three detection targets.

In the above-described aspect, the shape calculating portion may set the sections so as to include three or more of the adjacent detection targets each, and may obtain a polynomial expression of a curve representing the shape of the inserted portion in the individual sections by means of curve fitting based on the three-dimensional positions of the individual detection targets.

Another aspect of the present invention is a manipulator control method including: calculating a shape of a flexible inserted portion by dividing the inserted portion into a plurality of sections arranged in the longitudinal direction of the inserted portion; and controlling driving of a manipulator disposed at a distal end of the inserted portion by a drive portion disposed on the basal-end side of the inserted portion on the basis of the calculated shape of the inserted portion.

With this aspect, it is possible to calculate, with high precision, the shape of the inserted portion in which the overall shape thereof has been deformed in a complicated manner, even if estimation in each section is performed by using a simple shape. Also, it is possible to control, with high precision, driving of the manipulator at the distal end of the inserted portion by the drive portion disposed on the basal-end side of the inserted portion on the basis of the estimated shape of the inserted portion.

{Advantageous Effects of Invention}

The present invention affords an advantage in that, even when an inserted portion is bent in a complicated manner, it is possible to control a manipulator at the distal end thereof with high precision.

{Brief Description of Drawings}

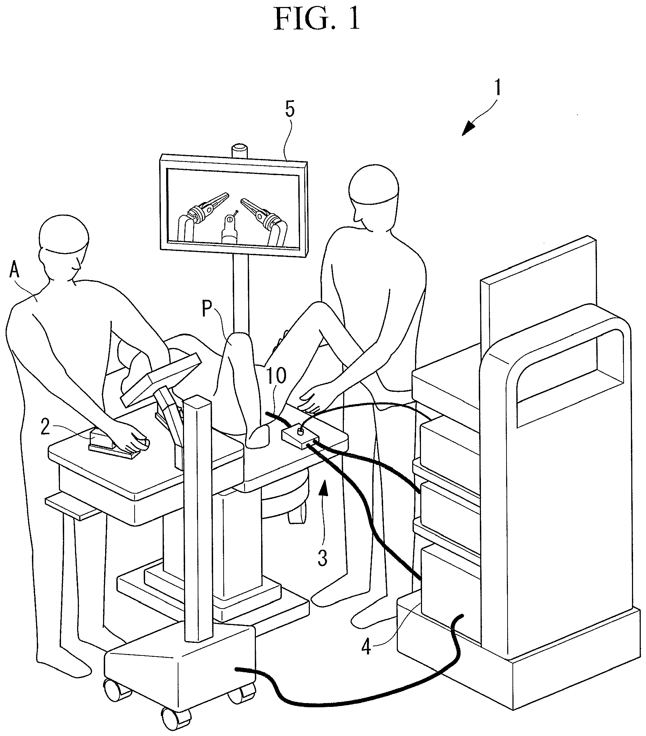

{Fig. 1} Fig. 1 is an overall configuration diagram showing a manipulator system according to an embodiment of the present invention.

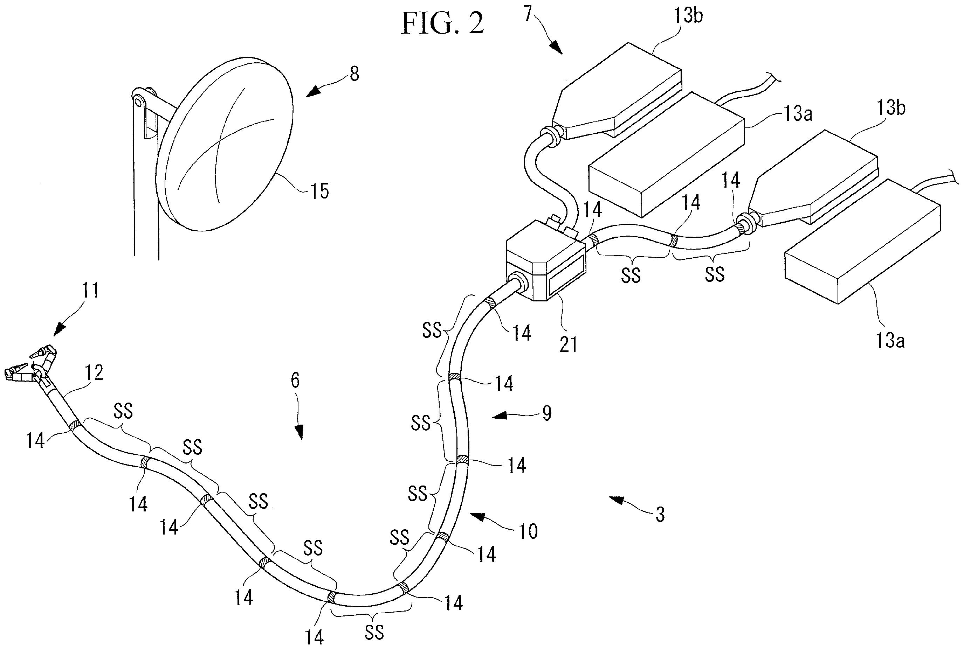

{Fig. 2} Fig. 2 is a perspective view showing a manipulator of the manipulator system in Fig. 1.

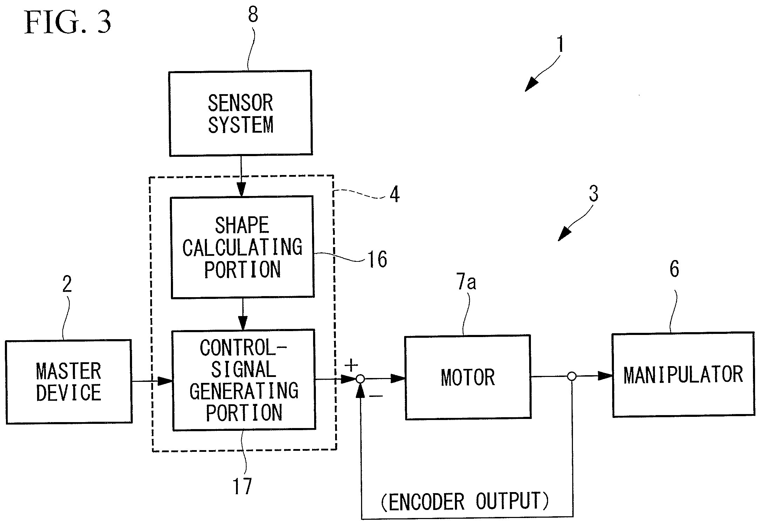

{Fig. 3} Fig. 3 is a block diagram for explaining the manner in which the manipulator in Fig. 2 is controlled.

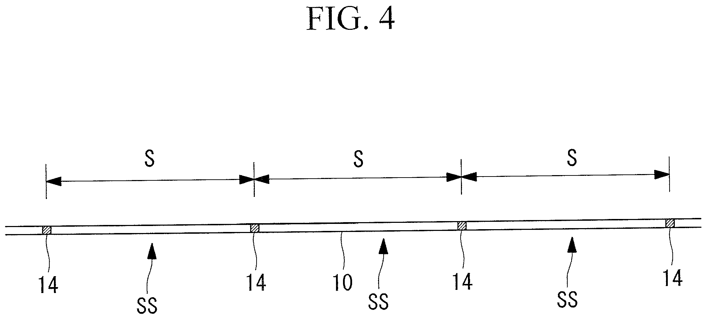

{Fig. 4} Fig. 4 is a diagram for explaining the arrangement of magnetic coils attached to a flexible portion of the manipulator in Fig. 2.

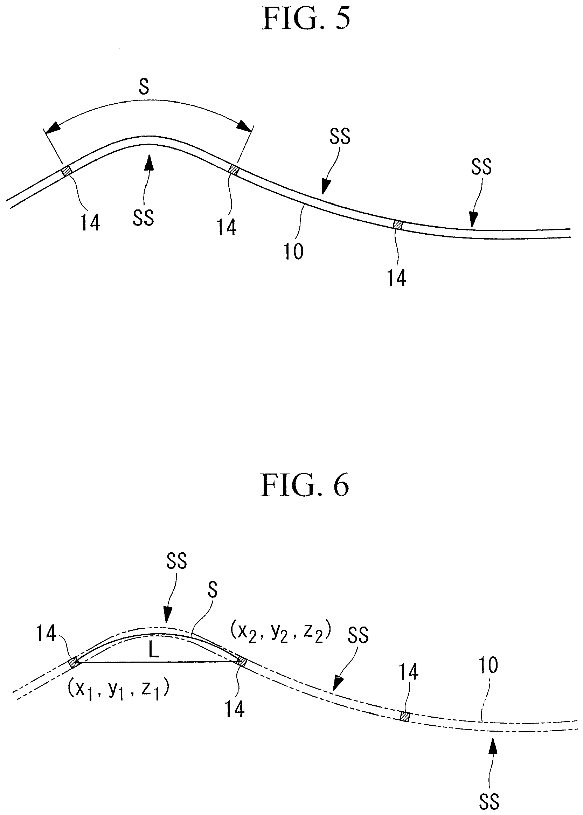

{Fig. 5} Fig. 5 is a diagram showing a state in which the flexible portion in Fig. 4 is bent.

{Fig. 6} Fig. 6 is a diagram for explaining the case in which the shape of one section of the flexible portion in Fig. 5 is estimated by using a single arc.

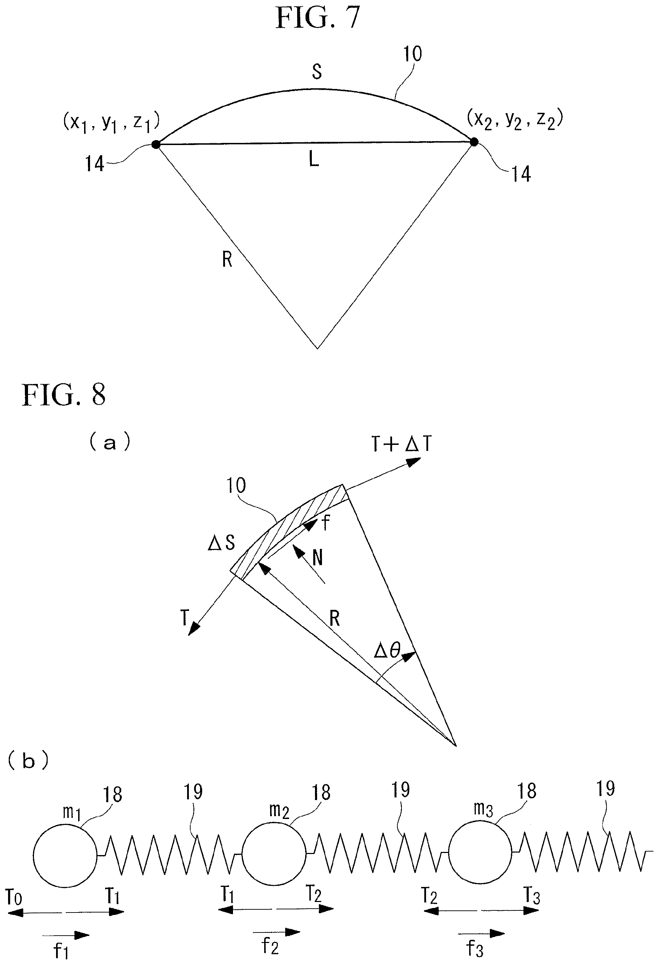

{Fig. 7} Fig. 7 is a schematic diagram of the section in which the shape thereof is estimated as shown in Fig. 6.

{Fig. 8} Fig. 8 includes (a) a diagram showing various values for developing a model for one section of the flexible portion in the manipulator system in Fig. 1, and (b) a diagram showing a wire model of the flexible portion.

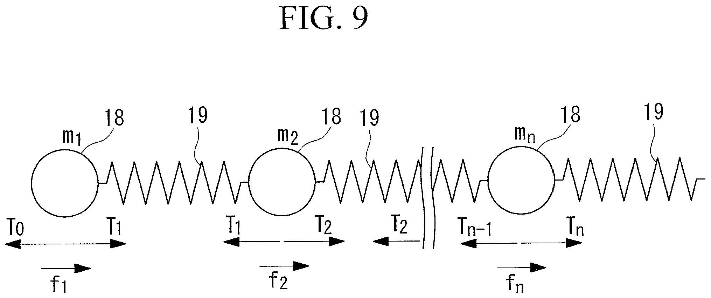

{Fig. 9} Fig. 9 is a diagram showing a wire model of the flexible portion, showing a modification of Fig. 8.

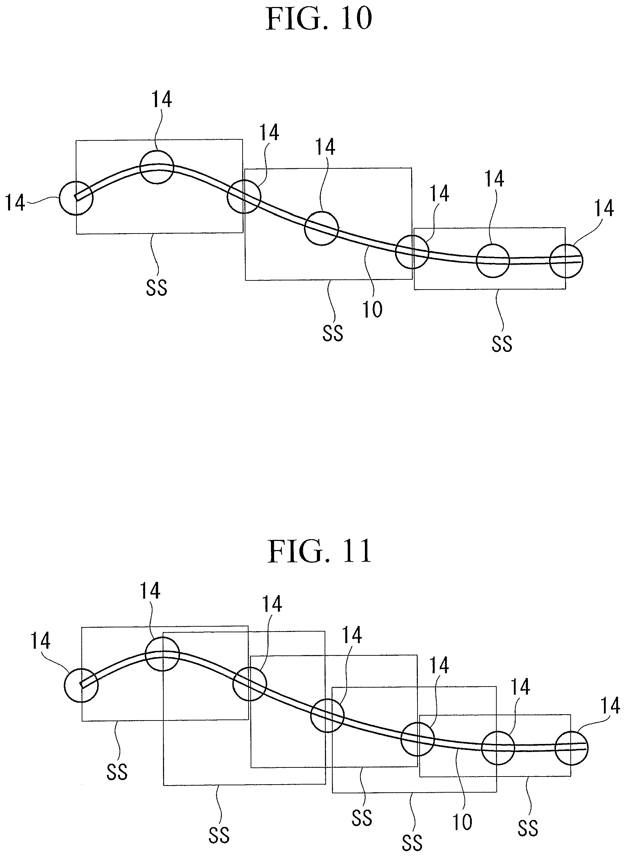

{Fig. 10} Fig. 10 shows a modification of the manipulator system in Fig. 1, and is a diagram for explaining estimation that involves sections including three magnetic coils each.

{Fig. 11} Fig. 11 shows a modification of the manipulator system in Fig. 1, and is a diagram for explaining estimation that involves overlapping sections.

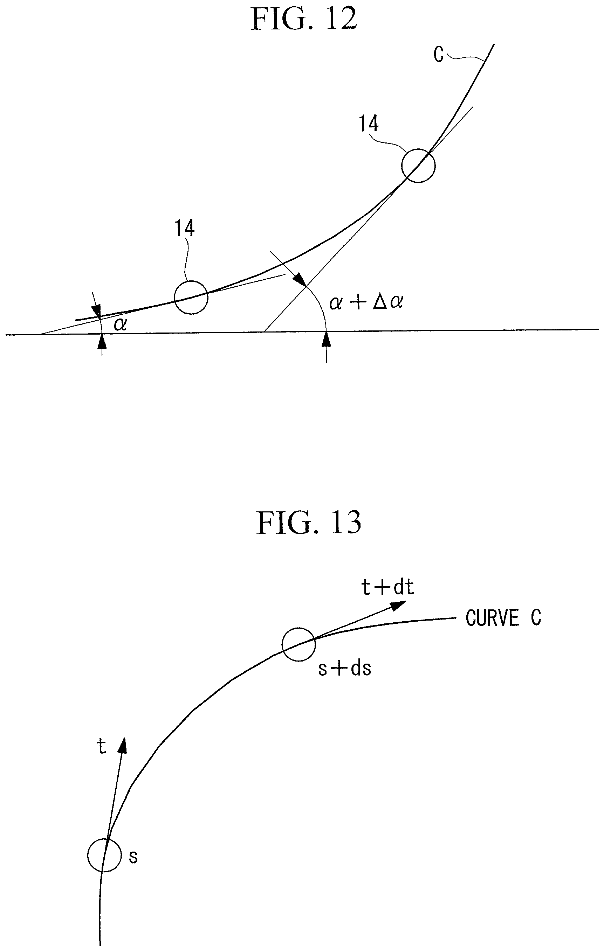

{Fig. 12} Fig. 12 is a diagram for explaining another method of estimating the shape of the flexible portion.

{Fig. 13} Fig. 13 is a diagram for explaining yet another method of estimating the shape of the flexible portion.

{Description of Embodiment}

A manipulator system 1 and a control method thereof according to an embodiment of the present invention will be described below with reference to the drawings.

As shown in Fig. 1, the manipulator system 1 according to this embodiment is provided with: a master device 2 that is manipulated by a doctor A; a slave device 3 that is driven in accordance with inputs given via the master device 2; a controller 4 that controls the slave device 3 on the basis of the inputs to the master device 2; and a monitor 5.

As shown in Fig. 2, the slave device 3 is provided with: a manipulator 6 according to this embodiment that is inserted into a body cavity of a patient P; a drive portion 7 that drives the manipulator 6; and a sensor system 8 that detects the shape of the manipulator 6.

The manipulator 6 is a flexible endoscope that has a flexible long, thin bendable inserted portion 9, and that is provided with: a long, thin flexible portion 10; a distal-end portion 11 disposed at the distal end thereof; and a bending portion 12 that is disposed between the distal-end portion 11 and the flexible portion 10.

The drive portion 7 is provided with: linear motion mechanisms 13a that are driven by motors 7a; connecting portions 13b that are attached to the linear motion mechanisms 13a in an attachable/detachable manner and that accommodate, in the interiors thereof, wires that connect the distal-end portion 11 and the connecting portions 13b; and a relaying portion 13c that consolidates the plurality of connecting portions 13b. The distal-end portion 11 is formed of, for example, arms having a plurality of joints, and the individual joints are driven by the pulling forces of the wires.

As shown in Fig. 3, the motors 7a of the drive portion 7 are driven on the basis of instruction signals transmitted thereto from the controller 4 and are controlled so as to achieve a driving level in accordance with the instruction signals by feeding back the outputs from encoders that are provided in the motors 7a and that detect the driving level.

The sensor system 8 is provided with: for example, a plurality of magnetic coils (detection targets) 14 that are provided, with spaces therebetween, in the flexible portion 10 in the longitudinal direction thereof; and an antenna (shape detecting portion) 15 that receives magnetic fields generated by the magnetic coils 14. By receiving the magnetic fields generated by the magnetic coils 14 by using the antenna 15, it is possible to acquire three-dimensional positional information of the individual magnetic coils 14. The three-dimensional positional information of the individual magnetic coils 14 acquired by the sensor system 8 is transmitted to the controller 4.

The controller 4 is provided with: a shape calculating portion 16 that calculates the shape of the flexible portion 10 on the basis of the three-dimensional positional information of the individual magnetic coils 14 that has been transmitted thereto; and a control-signal generating portion 17 that controls the drive portion 7 on the basis of the shape of the flexible portion 10 estimated by the shape calculating portion 16.

The sensor system 8 and the shape calculating portion 16 constitute a shape estimating portion.

The control-signal generating portion 17 outputs the amounts by which the wires are displaced in the longitudinal direction to the drive portion 7 as the instruction signals.

The operation of the shape calculating portion 16 provided in the controller 4 of the manipulator system 1 according to this embodiment will be described below.

As shown in Fig. 4, interval sizes S along the flexible portion 10 for the magnetic coils 14 provided in the flexible portion 10 are stored in the shape calculating portion 16 in advance. Thus, the shape calculating portion 16 sets a section SS for each set of two adjacent magnetic coils 14, and calculates the shape of the flexible portion 10 for each section SS.

As shown in Figs. 5 and 6, by using the three-dimensional positional information of the individual magnetic coils 14 acquired by the sensor system 8, a chord length L between the two adjacent magnetic coils 14 is calculated for all sets by using the expression below.

$$L = \sqrt{((x2 - x1)2 + (y2 - y1)2 + (z2 - z1)2)}$$

Here, only the two magnetic coils 14 at the distal end are shown in Figs. 5 and 6, and (x1, y1, z1) and (x2, y2, z2) are coordinate positions of each of the two adjacent magnetic coils 14.

Assuming that the flexible portion 10 that is bent as shown in Fig. 5 between the two adjacent magnetic coils 14 is evenly bent at a radius of curvature R, as shown in Figs. 6 and 7, it is possible to obtain the radius of curvature R by repeating calculations so that the Expression (1) below holds.

$$L - 2R\sin(S/2R) = 0 \qquad (1)$$

By doing so, the flexible portion 10 between all sets of two adjacent magnetic coils 14 is estimated in the form of arcs bent at the radius of curvature R.

In order to control the drive portion 7, the controller 4 is provided with an equation of motion described in Eq. 1 in accordance with a wire model shown in Fig. 8. In the example shown in Fig. 8(b), the equation of motion Eq. 1 holds assuming that the wire model is such that the individual sections SS are coupled, at mass points 18 having masses m, by using springs 19 having a predetermined stiffness. Here, an example in which the number of sections SS is three is described.

{Eq. 1}

$$\begin{pmatrix} m_1 & 0 & 0 \\ 0 & m_2 & 0 \\ 0 & 0 & m_3 \end{pmatrix} \begin{pmatrix} z''_1 \\ z''_2 \\ z''_3 \end{pmatrix} = -\begin{pmatrix} c_1 & 0 & 0 \\ 0 & c_2 & 0 \\ 0 & 0 & c_3 \end{pmatrix} \begin{pmatrix} z'_1 \\ z'_2 \\ z'_3 \end{pmatrix} + \begin{pmatrix} T_0 - T_1 - f_1 \\ T_1 - T_2 - f_2 \\ T_2 - T_3 - f_3 \end{pmatrix}$$

Here,

$m_1$, $m_2$, and $m_3$ are the masses of the wires in the individual sections SS (1 to 3),
$c_1$, $c_2$, and $c_3$ are viscous friction coefficients of the wires in the individual sections SS (1 to 3) in the longitudinal direction,
$z_1'$, $z_2'$, and $z_3'$ are moving speeds of the wires in the individual sections SS (1 to 3) in the longitudinal direction,
$z_1''$, $z_2''$, and $z_3''$ are accelerations of the wires in the individual sections SS (1 to 3) in the longitudinal direction,
$T_0$, $T_1$, $T_2$, and $T_3$ are tensile forces exerted on the wires in the individual sections SS (1 to 3), and
$f_1$, $f_2$, and $f_3$ are frictional forces in the individual sections SS (1 to 3).

In this equation of motion, frictional forces f (the individual frictional forces $f_1$, $f_2$, and $f_3$) are expressed by the following expression.

{Eq. 2}

$$f = sgn(z') \mu (\Delta S / R) T$$

Here,

sgn(z') is a function indicating the sign of the speed z', $\mu$ is the dynamic friction coefficient,
$\Delta S$ is the arc length,
R is the radius of curvature, and
T is the tensile force.

In Eq. 2, the value estimated by using Expression (1) is used as the radius of curvature R.

With this Eq. 2, the frictional forces f change in accordance with bending of the flexible portion 10 constituting the individual sections SS. Therefore, in the case in which the bending of the flexible portion 10 constituting the individual sections SS changes, the equation of motion stored in the controller 4 is corrected via changes in the frictional forces f.

The operation of the thus-configured manipulator system 1 according to this embodiment will be described below.

In order to treat an affected site located in the body cavity of the patient P by using the manipulator system 1 according to this embodiment, the manipulator 6 is introduced into the body cavity from the distal-end portions 11, and the distal-end portions 11 are made to face the affected site.

By doing so, the flexible portion 10 constituting the inserted portion of the manipulator 6 is bent in a complicated manner so as to conform to the shape of the body cavity. In this state, the antenna 15 detects the three-dimensional positions of the magnetic coils 14 mounted on the flexible portion 10 when the sensor system 8 is activated.

When the positional information of the individual magnetic coils 14, detected by the antenna 15, is transmitted to the controller 4, the shape calculating portion 16 provided in the controller 4 calculates the shape of the flexible portion 10.

In the manipulator system 1 according to this embodiment in this case, the shape calculating portion 16 performs calculations regarding the flexible portion 10 for each of the plurality of sections SS arranged in the longitudinal direction thereof.

In the example described in this embodiment, because the shape calculating portion 16 sets the sections SS to all sets of the two adjacent magnetic sensors 14 and the flexible portion 10 in the individual sections SS is estimated by using a single arc, it is possible to calculate the complicated shape of the flexible portion 10 with high precision by using simple estimations. Then, on the basis of the radius of curvature R of the flexible portion 10 estimated for each of the sections SS, the frictional forces f are calculated by using Eq. 2, and the equation of motion Eq. 1 is corrected by using the frictional forces f1, f2, and f3 calculated for each of the sections SS.

By solving the motion equation Eq. 1, the control-signal generating portion 17 calculates displacement levels A of the wires, which serve as the instruction signals, and outputs these signals to the drive portion 7 after converting the displacement levels A to driving levels of the motors for achieving these displacement levels A. By doing so, the motors 7a are driven at the instructed driving levels, as a result of which the wires are displaced by the desired displacement levels, and thus, the individual joints of the arms constituting the distal-end portion 11 are driven with high precision.

As has been described above, with the manipulator system 1 and the control method thereof according to this embodiment, there is an advantage in that, in the case in which the arms disposed at the distal end of the flexible portion 10 are driven by pulling the wires by means of the motors 7a disposed on the basal-end side of the flexible portion 10, it is possible to calculate, with high precision, the instruction signals for driving the joints of the arms by estimating the shape of the flexible portion 10 with high precision even when the shape of the flexible portion 10 is bent in a complicated manner. Also, because the flexible portion 10 is divided into the plurality of sections SS when estimating the shape thereof and the shape is estimated for each section SS in a simple manner by using a single arc, it is possible to reduce the amount of time required for calculation by using a simple calculation, thus making it possible to perform real-time control.

Note that, in this embodiment, although an example in which three sections SS are established has been described, any number of the sections SS may be established, as shown in Fig. 9 and Eq. 3.

{Eq. 3}

$$\begin{pmatrix} m_1 & 0 & \cdots & 0 \\ 0 & m_2 & \cdots & 0 \\ \vdots & \vdots & \ddots & \vdots \\ 0 & 0 & \cdots & m_n \end{pmatrix} \begin{pmatrix} z''_1 \\ z''_2 \\ \vdots \\ z''_n \end{pmatrix} = - \begin{pmatrix} c_1 & 0 & \cdots & 0 \\ 0 & c_2 & \cdots & 0 \\ \vdots & \vdots & \ddots & \vdots \\ 0 & 0 & \cdots & c_n \end{pmatrix} \begin{pmatrix} z'_1 \\ z'_2 \\ \vdots \\ z'_n \end{pmatrix} + \begin{pmatrix} T_0 - T_1 - f_1 \\ T_1 - T_2 - f_2 \\ \vdots \\ T_{n-1} - T_n - f_n \end{pmatrix}$$

In addition, in this embodiment, although a single arc is assumed for each section SS, and the radius of curvature R thereof is estimated, alternatively, a bending angle $\theta$ may be estimated.

In addition, because computation for estimating the radius of curvature R or the bending angle $\theta$ is time consuming, the computation may be omitted when the parts of the flexible portion 10 constituting the individual sections SS take straight-line shapes or a known shape, such as identical arcs.

For example, in the case in which the chord lengths L of the parts of the flexible portion 10 calculated in the form of the direct distance between the two magnetic coils 14 in the individual sections SS are equal to the distances between the magnetic coils 14 in the direction along the flexible portion 10, it is possible to consider those sections SS as having a straight-line shape, and thus, it is possible to omit the repeated computation for estimating the radius of curvature R or the like. In addition, in the case in which the radius of curvature R or the like is estimated in one of the sections SS, when the magnetic coils 14 of the adjacent section SS are arranged on that estimated arc, the radius of curvature R or the like that has already been estimated may be used instead of estimating the radius of curvature R or the like again.

In addition, the intervals between the magnetic coils 14 may be at equal distances or may be non-equal distances.

In addition, although each section SS is provided between two adjacent magnetic coils 14, and the radius of curvature R of the arc is estimated by setting the distance between the two magnetic coils 14 to be the chord length L, alternatively, as shown in Fig. 10, the radius of curvature R may be estimated by assuming that each section SS includes three or more magnetic coils 14 and by using an arc that passes through the positions of these magnetic coils 14. Note that the sections SS may be independent of each other, as shown in Fig. 10 or may overlap as shown in Fig. 11.

In addition, the shape of the flexible portion 10 may be constantly estimated in real time when the slave device 3 is being operated, or the estimation may manually be performed when the preparation for operating the slave device 3 is completed, periodically, or as needed.

In addition, as shown in Fig. 12, in this embodiment, a polynomial expression for a curve C may be obtained by means of curve fitting by using the positional information of the three or more magnetic coils 14, the tangent lines of the curve C at the positions of the individual magnetic coils 14 may be obtained, and the differences $\Delta\alpha$ between inclination angles $\alpha$ and $\alpha+\Delta\alpha$ of the adjacent tangent lines may be computed as bending angles. In this case, assuming that the arc length $\Delta S$ between the magnetic coils 14 is known, the radius of curvature R can be determined as below:

$$R = \Delta S / \Delta\alpha.$$

In addition, as shown in Fig. 13, the curve C representing the flexible portion 10 may be expressed by an arc length S of each section SS, and a curvature $\kappa$ may be determined, as in the following Expression, by differentiating a difference dt between tangent-line vectors t and t+dt by an arc length ds.

$$\kappa = |dt/ds|$$

In addition, in this embodiment, although an Endoscope Position Detecting Unit (UPD) constituted of the magnetic coils 14 and the antenna 15 provided in the flexible portion 10 has been described as the sensor system 8, alternatively, the shape of the flexible portion 10 after insertion may be predicted by using information on the shape of the organ obtained before the surgery, via CT, MRI, or the like. In addition, the shape may be detected by attaching a sensor that detects bending, such as a distortion sensor, an optical fiber sensor, or the like, to the flexible portion 10.

In addition, although the distal-end portions 11 having the arms have been described as examples of the joint portions at the distal end of the inserted portion 9, a flexible endoscope having no distal-end portion 11 may be employed. In this case, the bending portion 12 constitutes the joint portion at the distal end of the inserted portion.

In addition, although a flexible endoscope has been described as an example of the manipulator 6, a flexible treatment tool or a flexible over-tube may be employed.

{Reference Signs List}

SS section
1 manipulator system
6 manipulator
7 drive portion
10 flexible portion (inserted portion)
14 magnetic coil (detection target)
15 antenna (position detection portion, shape estimating portion)
16 shape calculating portion (shape estimating portion)
17 control-signal generating portion (control portion)

**Claims**

1. A manipulator system comprising:

a flexible inserted portion;
a manipulator that has a joint portion that is driven at a distal end of the inserted portion;
a drive portion that drives the joint portion of the manipulator;
a shape estimating portion that estimates the shape of the inserted portion; and
a control portion that controls the drive portion on the basis of the shape of the inserted portion estimated by the shape estimating portion,
wherein the shape estimating portion is provided with a plurality of detection targets that are arranged, with spaces therebetween, in the inserted portion in the longitudinal direction thereof, a position detection portion that detects three-dimensional positions of the individual detection targets, and a shape calculating portion that calculates, on the basis of the three-dimensional positions of the individual detection targets detected by the position detection portion, the shape of the inserted portion by dividing the inserted portion into a plurality of sections that are arranged in the longitudinal direction thereof.

2. A manipulator system according to Claim 1, wherein the shape calculating portion sets each of the sections so as to be located between two of the detection targets that are adjacent to each other.

3. A manipulator system according to Claim 1, wherein the shape calculating portion sets each of the sections so as to include three of the detection targets that are adjacent to each other.

4. A manipulator system according to Claim 2, wherein the shape calculating portion approximates, for each of the sections, a part between two of the detection targets that are adjacent to each other by using a single arc.

5. A manipulator system according to Claim 3, wherein the shape calculating portion approximates, for each of the sections, a part across three of the adjacent detection targets by using a single arc.

6. A manipulator system according to Claim 1, wherein the shape calculating portion sets the sections so as to include three or more of the adjacent detection targets each, and obtains a polynomial expression of a curve representing the shape of the inserted portion in the individual sections by means of curve fitting based on the three-dimensional positions of the individual detection targets.

7. A manipulator control method comprising:

calculating a shape of a flexible inserted portion by dividing the inserted portion into a plurality of sections arranged in the longitudinal direction of the inserted portion; and
controlling driving of a manipulator disposed at a distal end of the inserted portion by a drive portion disposed on the basal-end side of the inserted portion on the basis of the calculated shape of the inserted portion.

# FIG. 1

FIG. 2

FIG. 3

FIG. 4

# FIG. 5

# FIG. 6

# FIG. 7

# FIG. 8

(a)

(b)

EP 3 108 797 A1

# FIG. 9

# FIG. 10

# FIG. 11

# FIG. 12

# FIG. 13

## INTERNATIONAL SEARCH REPORT

| International application No. |
| --- |
| PCT/JP2015/054355 |

**A. CLASSIFICATION OF SUBJECT MATTER**
*A61B1/00*(2006.01)i, *G02B23/24*(2006.01)i

According to International Patent Classification (IPC) or to both national classification and IPC

**B. FIELDS SEARCHED**

Minimum documentation searched (classification system followed by classification symbols)
A61B1/00-1/32, G02B23/24-23/26

Documentation searched other than minimum documentation to the extent that such documents are included in the fields searched
| | | | |
| --- | --- | --- | --- |
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2007-159738 A (Olympus Medical Systems Corp.), 28 June 2007 (28.06.2007), paragraphs [0014] to [0021], [0105] to [0159]; fig. 1, 20 to 34 (Family: none) | 1-7 |
| Y | JP 2008-245839 A (Olympus Medical Systems Corp.), 16 October 2008 (16.10.2008), paragraphs [0014] to [0039]; fig. 1 to 3 & US 2010/0016666 A1 & WO 2008/120508 A1 & EP 2130479 A1 & CN 101610712 A | 1-7 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 01 May 2015 (01.05.15) | 19 May 2015 (19.05.15) |

| Name and mailing address of the ISA/ | Authorized officer |
| --- | --- |
| Japan Patent Office 3-4-3,Kasumigaseki,Chiyoda-ku, Tokyo 100-8915,Japan | Telephone No. |

Form PCT/ISA/210 (second sheet) (July 2009)

**INTERNATIONAL SEARCH REPORT**

International application No.

PCT/JP2015/054355

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2012/026184 A1  (Olympus Medical Systems Corp.),<br>01 March 2012 (01.03.2012),<br>paragraphs [0013] to [0139]; fig. 1 to 18<br>& JP 4897123 B1        & US 2012/0053412 A1<br>& EP 2460457 A1         & CN 102487602 A | 1-7 |
| Y | JP 2007-130175 A  (Pentax Corp.),<br>31 May 2007 (31.05.2007),<br>paragraphs [0013] to [0057]; fig. 1 to 6<br>& US 2007/0106114 A1     & DE 102006052886 A1 | 1-7 |
| A | JP 2002-238844 A  (Olympus Optical Co., Ltd.),<br>27 August 2002 (27.08.2002),<br>entire text; fig. 1 to 14<br>(Family: none) | 1-7 |
| A | WO 2007/080974 A1  (Olympus Medical Systems Corp.),<br>19 July 2007 (19.07.2007),<br>entire text; fig. 1 to 71<br>& JP 5134971 B1         & US 2007/0167679 A1<br>& EP 1982660 A1 | 1-7 |
| A | JP 2008-245838 A  (Olympus Medical Systems Corp.),<br>16 October 2008 (16.10.2008),<br>entire text; fig. 1 to 9<br>(Family: none) | 1-7 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009131406 A